# EUROPEAN PATENT SPECIFICATION

(11) **EP 0 779 267 B1**
(45) Date of publication and mention of the grant of the patent: **15.03.2000**
(21) Application number: 96309026.1
(22) Date of filing: 11.12.1996
(51) Int. Cl.: C07C 67/343, C07C 69/63

(54) **Process for the preparation of triethyl-3-bromopropane-1,1,1-tricarboxylate**
Verfahren zur Herstellung von Triethyl-3-Bromopropan-1,1,1-Tricarboxylat
Procédé de préparation du 3-bromopropane-1,1,1-tricarboxylate de triéthyle

(30) Priority: 12.12.1995 GB 9525398
(43) Date of publication of application: 18.06.1997
(73) Proprietor: GREAT LAKES (UK) LIMITED, Widnes, Cheshire WA8 8NS (GB)
(72) Inventor: Gledhill, Paul Adrian, The Assoc. Octel Comp. Ltd., South Wirral L65 4HF (GB); Pardue, Marc Vincent, The Assoc. Octel Comp. Ltd., South Wirral L65 4HF (GB); Cottrell, Timothy, The Assoc. Octel Comp. Ltd., South Wirral L65 4HF (GB); Clarke, Paul Derek, The Assoc. Octel Comp. Ltd., South Wirral L65 4HF (GB); Radley, Peter Michael, The Assoc. Octel Comp. Ltd, South Wirral L65 4HF (GB)
(74) Representative: Lyons, Andrew John

(56) References cited:
- EP-A- 0 718 272
- JOURNAL OF ORGANIC CHEMISTRY, vol. 44, no. 20, 14 September 1979, pages 3492-3496, XP000651851 PADGETT, H.C. ET AL.: "The Alkoxycarbonyl Moiety as a Blocking Group. A Generally Useful Variation of the Malonic Ester Synthesis"

## Description

Triethyl-3-bromopropane-1,1,1-tricarboxylate (TBTC) has uses as an intermediate in the preparation of purine derivatives (see for example EP 0 302 644 A2) and substituted malonate esters (see for example JOC, 44, 3492 (1979)).

The known alkylation process for the preparation of TBTC comprises first reacting triethyl methanetricarboxylate with sodium ethoxide, isolating the sodiotriethylmethane tricarboxylate thus formed and then reacting it with 1,2-dibromoethane (JOC, *44*, 3492 (1979)). The yields are 83% for the first stage and 91% for the second stage giving an overall yield from triethyl methanetricarboxylate of 76%. This procedure is unsatisfactory for the manufacture of TBTC on an industrial scale because it involves a two stage procedure with isolation of solid sodiotriethylmethane tricarboxylate as an intermediate and also because residual sodium ethoxide is sufficiently reactive to cause decarboxylation of the product with formation of undesirable impurities.

JOC, *58*, 898-903, (1993) describes alkylation of triethyl methanetricarboxylate by a bromo compound using dry potassium carbonate in DMF solvent.

EP-A-718272 (LONZA AG) discloses a reaction in which triethyl methanetricarboxylate is alkylated by dibromoethane in the presence of potassium carbonate.

It is an object of the present invention to provide a process for the preparation of TBTC from triethyl methanetricarboxylate which avoids the need to isolate an intermediate, which does not require the use of a reactive reagent such as sodium ethoxide and which enables TBTC to be obtained in improved yields as compared with the above mentioned known process.

According to the present invention, there is thus provided a process for the preparation of triethyl-3-bromopropane-1,1,1-tricarboxylate which comprises reacting triethyl methanetricarboxylate with an inorganic base and 1,2-dibromoethane characterised in that the reaction is carried out in the presence of dimethylformamide in admixture with one or more co-solvents.

Suitable inorganic bases includes alkali metal carbonates, hydroxides and hydrides such as for example sodium and potassium carbonates, potassium hydroxide and sodium hydride with potassium carbonate being particularly preferred. Where the inorganic base is insoluble in the organic solvent it is preferably in the form of fine suspension in the organic solvent with not more than 1.0% by weight having a particle size greater than 1 mm (by sieve). Particularly advantageous for use in the process according to the invention are fine grade potassium carbonate or other inorganic bases in which not more than 5%, preferably 0.3 to 5%, by weight has a particle size greater than 0.5mm and not more than 13%, preferably 1 to 13%, by weight has a particle size greater than 0.25 mm and not more than 30%, preferably 5 to 30%, by weight has a particle size greater than 0.1 mm.

Suitable organic co-solvents include ethers (e.g. diethyleneglycol dimethylether, methyl tertiarybutyl ether and tetrahydrofuran), esters (e.g. ethyl acetate), ketones (e.g. acetone), hydrocarbons (e.g. toluene) and mixtures thereof. An excess of the 1,2-dibromoethane reactant can also if desired provide at least part of the organic solvent requirement. Dimethylformamide is used in admixture with one or more other solvents. It has been found that combining a dipolar aprotic solvent (particularly dimethylformamide) with a further solvent (such as an ether or ester) gives enhanced yields in the reaction over a dipolar aprotic solvent alone.

For 1 mole of triethyl methanetricarboxylate, it is preferable to use from 1.0 to 2.0 moles, more preferably from 1.0 to 1.75 moles and most preferably from 1.1 to 1.6 moles, of the inorganic base; and from 2.0 to 3.0 moles and more preferably from 2.0 to 2.5 moles of the 1,2-dibromoethane. At least 200 ml of DMF is preferably used for each mole of triethyl methanetricarboxylate together with up to 200 ml or more per mole of triethyl methanetricarboxylate of one or more other solvents.

The reaction temperature is conveniently in the range of from ambient temperature up to 150°C. At temperatures in the lower part of this range, longer reaction times will be necessary so that for example as long as 20 hours or more may be required at 50°C. At higher temperatures much shorter reaction times will be required so that for example at 90°C less than 1 hour may be sufficient but the use of higher temperatures may increase the formation of undesired by-products.
Preferably the reaction is carried out at temperatures within the range of from 40 to 90°C, more preferably from 50 to 80°C, with total reaction times of from 1 to 30 hours and more preferably from 5 to 25 hours.

It is advantageous to allow the triethyl methanetricarboxylate time to react with the inorganic base before addition of the 1,2-dibromoethane to the reaction mixture. It is thus advantageous first to contact the triethyl methanetricarboxylate with the inorganic base and then, preferably after a period of from 15 minutes to 3 hours and more preferably 30 minutes to 2 hours, to add the 1,2-dibromoethane to the reaction mixture. Prior to addition of the 1,2-dibromoethane, the reaction temperature is preferably held within the range of from 40 to 70°C with somewhat higher temperatures, for example within the range of from 50 to 80°C, then being used for the subsequent reaction with 1,2-dibromoethane.

The reaction can if desired by carried out in the presence of a phase transfer catalyst such as for example triethylbenzyl ammonium chloride or bromide, tetra-n-butyl ammonium bromide, tetra-n-octyl ammonium bromide, stearyl-tributyl phosphonium bromide or Adogen 464 (a commecially available tri-(C₈₋₁₀)-alkylmethyl ammonium chloride).

In a preferred method of carrying out the reaction, the triethyl methanetricarboxylate is added to a suspension of the inorganic base in the solvent mixture and then 1,2-dibromoethane is simultaneously or consecutively added. When the reaction is complete, the TBTC can be isolated by removal of inorganic salts, e.g. by filtration or washing with water, followed by evaporation and if necessary by distillation of the product.

The following examples illustrate the invention:

### Example 1

To a mixture of 700 ml dimethylformamide and 700 ml methyltertiarybutylether (MTBE) was added 578.8g fine grade potassium carbonate (4.19 moles). The stirred mixture was heated to 50°C and 848.8g of triethyl methanetricarboxylate (3.49 moles) was added in one portion. The temperature was allowed to rise to 65°C and then reduced to 50°C over one hour. The mixture was heated to 60°C and 1312g of 1,2-dibromoethane was added in one portion. The mixture was stirred at 60°C for 6 hours by which time gas chromatographic analysis of the reaction mixture showed that the reaction had come to an end. The mixture was cooled to ambient temperature, and 2800 ml of water was added. The organic phase was separated, the aqueous phase washed with 700 ml of MTBE and the organic phases combined. Solvents were removed by distillation to give 1255g of oil containing 1146.6g of TBTC, 97% yield contained. The material was distilled to give 84% yield of material at 97.6% assay.

### Examples 2-16

These examples were carried out following the procedure of Example 1. The solvent system was varied in each example. The contained yield of product before distillation was determined by quantitative analysis and is indicated in Table 1.

| Example | Solvent 1 | Solvent 2 | Ratio solvent 1: solvent 2 (volume) | Contained yield % |
|---|---|---|---|---|
| 2 | DMF | MTBE | 1:1 | 95 |
| 3 | DMF | MTBE | 1:1 | 91 |
| 4 | DMF | MTBE | 1:1 | 90 |
| 5 | DMF | MTBE | 1:1 | 90 |
| 6 | DMF | Ethyl acetate | 1:1 | 97 |
| 7 | DMF | Ethyl acetate | 1:1 | 90 |
| 8 | DMF | Tetrahydrofuran | 1:1 | 91 |
| 9 | DMF | Tetrahydrofuran | 1:1 | 91 |
| 10 | DMF | Tetrahydrofuran | 1:1 | 92 |
| 11 | DMF | Tetrahydrofuran | 1:1 | 94 |
| 12* | DMF | None | | 80 |
| 13* | DMF | None | | 71 |
| 14* | DMF | None | | 89 |
| 15* | DMF | None | | 82 |
| 16* | DMF | None | | 89 |

| | | | | |
|---|---|---|---|---|
| comparative examples | | | | |

The results show that the contained yield where DMF is used together with a second solvent (examples 2-11) is higher than when DMF is used alone (12-16).

## Claims

1. A process for the preparation of triethyl-3-bromopropane-1,1,1-tricarboxylate comprising the steps of reacting triethyl methanetricarboxylate with an inorganic base and 1,2-dibromoethane characterised in that the reaction is carried out in the presence of dimethylformamide in admixture with one or more co-solvents.

2. A process as claimed in claim 1 wherein said co-solvent is an ether or ester.

3. A process as claimed in claim 2 wherein said one or more co-solvents are selected from the group consisting of MTBE, ethyl acetate and THF.

4. A process as claimed in any preceding claim carried out in the presence of a phase transfer catalyst.

5. A process as claimed in any preceding claim wherein said base is fine grade potassium carbonate.

## Patentansprüche

1. Verfahren zur Herstellung von Triethyl-3-brompropan-1,1,1-tricarboxylat, das die Schritte der Reaktion von Triethyl-methantricarboxylat mit einer anorganischen Base und 1,2-Dibrommethan umfaßt, dadurch gekennzeichnet, daß die Reaktion in Gegenwart von Dimethylformamid unter Beimischung eines oder mehrerer weiterer Lösungsmittel ausgeführt wird.

2. Verfahren nach Anspruch 1, worin das weitere Lösungsmittel ein Ether oder ein Ester ist.

3. Verfahren nach Anspruch 2, worin das oder weitere Lösungsmittel ausgewählt sind aus der Gruppe bestehend aus MTBE, Ethylacetat und THF.

4. Verfahren nach einem der vorangehenden Ansprüche, ausgeführt in Gegenwart eines Phasentransfer-Katalysators.

5. Verfahren nach einem der vorangehenden Ansprüche, worin die Base feinkörniges Kaliumcarbonat ist.

## Revendications

1. Procédé pour la préparation de 3-bromopropane-1,1,1-tricarboxylate de triéthyle, comprenant les étapes de réaction de méthanetricarboxylate de triéthyle avec une base minérale et du 1,2-dibromoéthane, caractérisé en ce que la réaction est mise en oeuvre en présence de diméthylformamide en mélange avec un ou plusieurs co-solvants.

2. Procédé selon la revendication 1, dans lequel ledit co-solvant est un éther ou ester.

3. Procédé selon la revendication 2, dans lequel ledit ou lesdits co-solvants sont choisis dans l'ensemble constitué par le MTBE, l'acétate d'éthyle et le THF.

4. Procédé selon l'une quelconque des revendications précédentes, mis en oeuvre en présence d'un catalyseur de transfert de phase.

5. Procédé selon l'une quelconque des revendications précédentes, dans lequel ladite base est du carbonate de potassium de qualité fine.
